(19) Europäisches Patentamt
European Patent Office
Office européen des brevets

(11) EP 2 357 025 A2

(12) **EUROPEAN PATENT APPLICATION**

(43) Date of publication:
**17.08.2011 Bulletin 2011/33**

(51) Int Cl.:
*A61Q 19/00* (2006.01)     *A61K 8/06* (2006.01)
*A61K 8/31* (2006.01)     *A61K 8/368* (2006.01)
*A61K 8/37* (2006.01)     *A61K 8/58* (2006.01)
*A61K 8/92* (2006.01)

(21) Application number: **10252134.1**

(22) Date of filing: **16.12.2010**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **17.12.2009 US 640168**

(71) Applicant: **Johnson & Johnson Consumer
Companies, Inc.
Skillman, NJ 08558 (US)**

(72) Inventors:
• **Gunn, Euen T.
Trenton, NJ 08611-1723 (US)**
• **Bonner, Patricia
Annandale, NJ 08801 (US)**
• **Santora, Delores
Ringoes, NJ 08551 (US)**

(74) Representative: **Carpmaels & Ransford
One Southampton Row
London
WC1B 5HA (GB)**

(54) **Mild leave-on skin care compositions**

(57)     This invention relates to a composition that is mild to the skin containing a cosmetically acceptable oil; water; a cosmetically acceptable emulsifier having an HLB of from about 1 to about 25; and a preservative comprising an organic acid selected from the group consisting of benzoic acid, p-anisic acid, sorbic acid, lactic acid, acetic acid, formic acid, oxalic acid, tartaric acid, salicylic acid and citric acid; wherein said composition has a pH less than 5 and a buffer capacity of between about 0.001 and about 0.039.

EP 2 357 025 A2

**Description**

Cosmetic products designed to be applied and left on the skin typically have ingredients that include preservative systems engineered to be mild to the skin. In order to achieve such mildness, such products usually contain a preservative that exhibits preservative (microbial and fungal) activity at a physiological pH range (about 5.5 to about 6.5). To maintain the pH and the mildness, the compositions also generally contain a buffering system as well. As cosmetic products typically have a long shelf life, the buffering system may play an important role in the balance of preservative efficacy and mildness.

The pH of normal, healthy human skin is usually between about 4.5 and about 6. However, this pH may vary with age. Typically, the pH of newborn skin is closer to neutral (pH 7) and quickly turns acidic with time, in order to protect young children's skin.

Eye mildness of leave-on compositions is an important consideration when formulating products that are intended for application to the skin. Nonetheless, eyes have a "defense mechanism" that permits the adjustment of pH when a foreign composition contacts the eye. When a solution is added to the eye, tears quickly adjust the pH of the added solution and prevent prolonged stinging. The skin, however, has no such reservoir that acts to respond to a composition left on the skin. The result may be skin irritation and redness.

Therefore, it is desired to find a cosmetic or personal care leave-on formulation that is storage-stable, yet is designed so as to adjust quickly to the pH of the skin. This composition would have a low buffer capacity and a low pH in order to be compatible with the skin and would be storage-stable over a significant time period.

## SUMMARY OF THE INVENTION

The buffered, leave-on compositions of this invention comprise, consist essentially of and consist of: an oil; water; an emulsifier; and a preservative, which is an organic acid selected from the group consisting of benzoic acid, p-anisic acid, sorbic acid, lactic acid, acetic acid, formic acid, oxalic acid, tartaric, salicylic and citric acid, wherein the composition has a pH less than 5 and a buffer capacity of between 0.001 and 0.039.

## DETAILED DESCRIPTION OF THE INVENTION

A buffered composition is a composition that is able to retain an almost constant pH when either an acid or base is added. The quantitative measure of this resistance to pH changes is called "buffer capacity". As used herein, the term "buffer capacity" is the amount of acid or base the buffer can neutralize before the pH of the composition begins to change to a significant degree. Compositions applied to the skin react to the pH of the skin and may undergo a shift in pH. Thus, it is desired that the compositions of this invention be capable of "buffering" the effects of skin pH so as to maintain their characteristics, especially their mildness. The pH of compositions of this invention are preferably less than 5. More preferably, the pH of compositions of this invention are less than about 4.8.

The buffer capacity of the compositions is between about 0.001 and about 0.039, preferably from about 0.001 to about 0.031, more preferably from about 0.001 to about 0.023 as determined by the procedure set forth in the examples.

As used herein, the term "organic acid" means a carbon-containing compound having acidic properties (proton-donor). The most common organic acids are the carboxylic acids whose acidity is associated with their carboxyl group -COOH.

Examples of suitable organic acids for use in cosmetic compositions include benzoic acid, p-anisic acid, sorbic acid, lactic acid, acetic acid, formic acid, oxalic acid, tartaric acid, citric acid, salicylic acid, and the like.

Preferably the composition is preserved and buffered with an organic acid selected from potassium sorbate, sodium benzoate and benzoic acid.

The compositions of this invention preferably have a pH below 5. Because the composition is preserved with an organic acid, the acidic pH prevents the organic acid from totally converting into a salt. More preferably, the pH of the compositions of this invention should be 4.8 or below. For example, if the composition is initially preserved with benzoic acid at a pH of 4.8, raising the pH above 5 converts the benzoic acid into sodium benzoate.

The compositions of this invention preferably are in the form of at least a two-phase composition. More preferably, they are in the form of an oil-in-water emulsion.

The compositions of this invention preferably are in the form of at least a two-phase composition. More preferably, they are in the form of an oil-in-water emulsion.

The topical compositions useful in the compositions of this invention are preferably formulated as emulsions. If the carrier is an emulsion, from about 1% to about 10% (more preferably, from about 2% to about 5%) of the carrier is one or more emulsifier(s). Emulsifiers may be nonionic, anionic or cationic. Suitable emulsifiers are disclosed in, for example, U.S. Pat. Nos. 3,755,560, 4,421,769, McCutcheon's Detergents and Emulsifiers, North American Edition, pp. 317-324 (1986), and the ICI Handbook, pp.1673-1686.

Single emulsion skin care preparations, such as lotions, of the oil-in-water type and water-in-oil type are well-known in the cosmetic art and are useful in the compositions of this invention. Multiphase emulsion compositions, such as the water-in-oil-in-water type, as disclosed in U.S. Pat. Nos. 4,254,105 and 4,960,764, are also useful in the subject invention. In general, such single or multiphase emulsions contain water, emollients, and emulsifiers as essential ingredients.

Most preferably, the compositions of this invention are in the form of a lotion.

Lotions typically contain from about 1% to about 20% (more preferably, from about 5% to about 10%) of an emollient(s) and from about 50% to about 90% (more preferably, from about 60% to about 80%) of water.

Emulsifiers that are cosmetically acceptable and have appropriate hydrophilic-lipophilic balance ("HLB") are preferred for use in the compositions and methods of this invention. The Hydrophilic-lipophilic balance of a surfactant or emulsifier is a measure of the degree to which it is hydrophilic or lipophilic, determined by calculating values for the different regions of the molecule. Preferably, the HLB of emulsifiers useful in the compositions of this invention are from about 1 to about 25. More preferably, the HLB should be between about 2 and about 16. Most preferably, the HLB should be between about 4 and about 13. Emollients preferred for use in the compositions of this invention include the following: Potassium Cetyl Phosphate, Hydrogenated Palm Glycerides, Glyceryl Laurate, Candelilla/Jojoba/Rice Bran Polyglyceryl-3 Esters, Sodium Stearoyl Lactylate, Ceteareth-6, Polysorbate 61, Glyceryl Stearate, Polysorbate 20. More preferably, emulsifiers that may be used in the compositions of this invention include: Potassium Cetyl Phosphate, Hydrogenated Palm Glycerides, Glyceryl Laurate, Candelilla/Jojoba/Rice Bran Polyglyceryl-3 Esters, Sodium Stearoyl Lactylate, Ceteareth-6, Polysorbate 61, Glyceryl Stearate, Polysorbate 20. Most preferably, Glyceryl Laurate, Candelilla/Jojoba/Rice Bran Polyglyceryl-3 Esters, Sodium Stearoyl Lactylate. should be included in the compositions of this invention.

The oil phase of the compositions of this invention should also contain at least one cosmetically acceptable oil. As used herein, the term "oil" is a hydrophobic material that can aid in balancing the intermolecular forces to form micelle aggregates or to limit their sizes. Oils also serve as emollient ingredients to benefit product spreadability, skin feel and delivery of hydrophobic active ingredients such as but not limited to, Vitamins D, E, K and A, and sunscreen filters.

Oils that are useful in the compositions of this invention include a variety of hydrocarbon-based oil, silicones, fatty acid derivatives, glycerides, vegetable oils, vegetable oil derivatives, alkyl esters, wax esters, beeswax derivatives, sterols, and phospholipids and combinations thereof ranging from approximately 20% to 50%, based on the total weight of the composition.

Suitable hydrocarbon oils for preferable use in the compositions and methods of this invention include petrolatum, mineral oil, micro-crystalline waxes, squalene and combinations thereof. The example of silicone oils suitable for use as hydrophobic materials for this invention include dimethicone, dimethiconol, phenyl dimethicone and cyclic polysiloxanes and combinations thereof. Silicone oils having viscosities from about 0.5 to about 100,000 centistokes at 25°C may also be useful in the composition.

Glycerides useful in the compositions of this invention include castor oil, sunflower seed oil, coconut oil and derivatives, vegetable oils and derivatives, palm oil, jojoba oil, shea butter, lanolin and combinations thereof.

Alkyl ester oils including, but not limited to isopropyl esters of fatty acids and esters of long chain fatty acids may also be suitable for use in the compositions of this invention. More preferably, the following alkyl esters may be useful in the compositions of this invention: isopropyl palmitate, isopropyl myristate, myristyl myristate, isohexyl palmitate, decyl oleate, isononyl isononanoate and a combination thereof.

The compositions of this invention should also contain water. The water may also contain structuring agents such as carbomers or other thickening polymers, for example, xanthan gum, carageenan gum or the like.

The compositions may be made into a wide variety of product types that include but are not limited to lotions, sprays, wipes, and make-up such as foundations. These product types may comprise several types of cosmetically-acceptable topical carriers. Additional components may be included in the composition including benefit agents.

Examples of suitable benefit agents include, but are not limited to, depigmentation agents; reflectants; film forming polymers; humectants; amino acids and their derivatives; antimicrobial agents; allergy inhibitors; anti-acne agents; anti-aging agents; anti-wrinkling agents, antiseptics; analgesics; antitussives; antipruritics; local anesthetics; anti-hair loss agents; hair growth promoting agents; hair growth inhibitor agents, antihistamines such as *Mandragora Vernalis, Tanacetum Parthenium* and the like; antiinfectives such as *Acacia Catechu, Aloe Barbadensis, Convallaria Majalis, Echinacea, Eucalyptus, Mentha Piperita, Rosa Canina, Sassafras Albidum,* and the like; inflammation inhibitors; anti-emetics; anticholinergics; vasoconstrictors; vasodilators; wound healing promoters; peptides, polypeptides and proteins; deodorants and antiperspirants; medicament agents; skin emollients and skin moisturizers; skin firming agents, vitamins; tanning agents; skin lightening agents; antifungals such as *Centaurea Cyanus, Kalmia Latifolia* and antifungals for foot preparations; depilating agents; external analgesics; perfumes; counterirritants; hemorrhoidals; insecticides; poison ivy products; poison oak products; burn products; anti-diaper rash agents; prickly heat agents; make-up preparations; vitamins; amino acids and their derivatives; herbal extracts; retinoids; flavenoids; sensates; anti-oxidants; skin conditioners; hair lighteners; chelating agents; cell turnover enhancers; coloring agents; pigments; sunscreens, those active ingredients disclosed in U.S. Pat. No. 6,063,397, which is incorporated herein by reference, anti-edema agents,

3

collagen enhancers, and mixtures thereof.

Examples of suitable anti-edema agents nonexclusively include bisabolol natural, synthetic bisabolol, and mixtures thereof.

Examples of suitable vasoconstrictors nonexclusively include horse chestnut extract, prickly ash, and mixtures thereof.

Examples of suitable anti-inflammatory agents nonexclusively include benoxaprofen, *centella asiatica,* bisabolol, feverfew (whole), feverfew (parthenolide free), green tea extract, green tea concentrate, hydrogen peroxide, lycopene including "Lyc-o-Pen" available from LycoRed Natural Products Industries, Ltd., oat oil, *chamomile,* and mixtures thereof.

Examples of collagen enhancers nonexclusively include vitamin A, vitamin C, and mixtures thereof.

Examples of suitable skin firming agent nonexclusively include dimethylaminoethanol ("DMAE").

Examples of suitable antipruritics and skin protectants nonexclusively include oatmeal, betaglucan, feverfew, soy and derivatives thereof, bicarbonate of soda, colloidal oatmeal, surfactant based colloidal oatmeal cleanser, *Anagallis Arvensis, Oenothera Biennis, Verbena Officinalis,* and the like. These antipruritics may be used in an amount, based upon the total weight of the cleansing composition, from about 0.01 percent to about 40 percent, and preferably from about 1 percent to about 5 percent.

As used herein, colloidal oatmeal means the powder resulting from the grinding and further processing of whole oat grain meeting United States Standards for Number 1 or Number 2 oats. The colloidal oatmeal has a particle size distribution as follows: not more than 3 percent of the total particles exceed 150 micrometers in size and not more than 20 percent of the total particles exceed 75 micrometers in size. Examples of suitable colloidal oatmeals include, but are not limited to, "Tech-O" available from the Beacon Corporation and colloidal oatmeals available from Quaker.

Examples of suitable reflectants nonexclusively include mica, alumina, calcium silicate, glycol dioleate, glycol distearate, silica, sodium magnesium fluorosilicate, and mixtures thereof.

Suitable film forming polymers include those that, upon drying, produce a substantially continuous coating or film on the hair, skin, or nails. Nonexclusive examples of suitable film forming polymers include acrylamidopropyl trimonium chloride/acrylamide copolymer; corn starch/acrylamide/sodium acrylate copolymer; polyquaternium-10; polyquaternium-47; polyvinylmethylether/maleic anhydride copolymer; styrene/acrylates copolymers; and mixtures thereof.

Commercially available humectants which are capable of providing moisturization and conditioning properties are suitable for use in the present invention. The humectant is preferably present in an amount of from about 0 percent to about 10 percent, more preferably from about 0.5 percent to about 5 percent, and most preferably from about 0.5 percent to about 3 percent, based on the overall weight of the composition. Glycerin is an example of a humectant.

Suitable amino acid agents include amino acids derived from the hydrolysis of various proteins as well as the salts, esters, and acyl derivatives thereof. Examples of such amino acid agents nonexclusively include amphoteric amino acids such as alkylamido alkylamines, i.e. stearyl acetyl glutamate, capryloyl silk amino acid, capryloyl collagen amino acids; capryloyl keratin amino acids; capryloyl pea amino acids; cocodimonium hydroxypropyl silk amino acids; corn gluten amino acids; cysteine; glutamic acid; glycine; hair keratin amino acids; amino acids such as aspartic acid, threonine, serine, glutamic acid, proline, glycine, alanine, cystine, valine, methionine, isoleucine, leucine, tyrosine, phenylalanine, cysteic acid, lysine, histidine, arginine, cysteine, tryptophan, citrulline; lysine; silk amino acids, wheat amino acids; and mixtures thereof.

Suitable proteins include those polymers that have a long chain, i.e. at least about 10 carbon atoms, and a high molecular weight, i.e. at least about 1000, and are formed by self-condensation of amino acids. Nonexclusive examples of such proteins include collagen, deoxyribonuclease, iodized corn protein; milk protein; protease; serum protein; silk; sweet almond protein; wheat germ protein; wheat protein; alpha and beta helix of keratin proteins; hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof.

Examples of suitable vitamins nonexclusively include vitamin B complex; including thiamine, nicotinic acid, biotin, pantothenic acid, choline, riboflavin, vitamin B6, vitamin B12, pyridoxine, inositol, carnitine; vitamins A, C, D, E, K and their derivatives such as vitamin A palmitate and pro-vitamins, e.g. (i.e. panthenol (pro vitamin B5) and panthenol triacetate) and mixtures thereof.

Examples of suitable antibacterial agents nonexclusively include bacitracin, erythromycin, neomycin, tetracycline, chlortetracycline, benzethonium chloride, phenol, and mixtures thereof.

Examples of suitable cosmetically acceptable skin emollients and skin moisturizers nonexclusively include mineral oil, lanolin, plant-derived oils including but not limited to cocoglycerides, coconut oil, palm kernel oil, babssu oil, sunflower seed oil, japan wax, palm oil, apricot kernel oil, tallow, argan oil, baobab oil, cocoa butter, andiroba seed oil, mango butter, avocado oil, cottonseed oil, rice bran oil, shea butter, marula oil, papaya seed oil, pumpkin seed oil, wheat germ oil, illipe butter, corn oil, olive oil, poppy seed oil, grapeseed oil, sesam oil, yangu seed oil, sweet almond oil, hazelnut oil, soybean oil, acai oil, safflower oil, hydbrid safflower oil, walnut oil, canola oil, black currant seed oil, hazel seed oil, peanut oil, cranberry seed oil, tall oil, kokum butter, manketti nut oil, moringa oil, raspberry seed oil, cupuacu butter, linseed oil, tung oil, jojoba oil, borage seed oil, evenining primrose oil, veronica oil, ongokea oil], vegetable oils, isostearyl isostearate, glyceryl laurate, methyl gluceth-10, methyl gluceth-20 chitosan, and mixtures thereof.

Examples of sunscreen agents nonexclusively include benzophenones, bornelone, butyl paba, cinnamidopropyl trimethyl ammonium chloride, disodium distyrylbiphenyl disulfonate, paba, potassium methoxycinnamate, butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, Padimate O, red petrolatum, and mixtures thereof.

An example of a suitable tanning agent nonexclusively includes dihydroxyacetone.

Examples of skin lightening agents nonexclusively include hydroquinone, catechol and its derivatives, ascorbic acid and its derivatives, and mixtures thereof.

Examples of suitable insecticides (including insect repellents, anti-scabies and anti-lice treatments) nonexclusively include permethrin, pyrethrin, piperonyl butoxide, imidacloprid, N,N-diethyl toluamide, which refers to the material containing predominantly the meta isomer, i.e., N,N-diethyl-m-toluamide, which is also known as DEET and other materials.

An example of an anti fungal for foot preparations nonexclusively includes tolnaftate.

Examples of suitable depilatory agents nonexclusively include calcium thioglycolate, magnesium thioglycolate, potassium thioglycolate, strontium thioglycolate, and mixtures thereof.

Examples of suitable external analgesics and local anesthetics nonexclusively include benzocaine, dibucaine, benzyl alcohol, camphor, capsaicin, *capsicum, capsicum* oleoresin, juniper tar, menthol, methyl nicotinate, methyl salicylate, phenol, resorcinol, turpentine oil, and mixtures thereof.

Examples of suitable antiperspirants and deodorants nonexclusively include aluminium chlorohydrates, aluminium zirconium chlorohydrates, and mixtures thereof.

Examples of suitable counterirritants nonexclusively include camphor, menthol, methyl salicylate, peppermint and clove oils, ichtammol, and mixtures thereof.

An example of a suitable inflammation inhibitor nonexclusively includes hydrocortisone, *Fragaria Vesca, Matricaria Chamomilla,* and *Salvia Officinalis.*

Examples of suitable hemorrhoidal products nonexclusively include the anesthetics such as benzocaine, pramoxine hydrochloride, and mixtures thereof; antiseptics such as benzethonium chloride; astringents such as zinc oxide, bismuth subgallate, balsam Peru, and mixtures thereof; skin protectants such as cod liver oil, vegetable oil, and mixtures thereof.

Most preferred benefit agents nonexclusively include DMAE, soy and derivatives thereof, colloidal oatmeal, sulfonated shale oil, olive leaf, elubiol, 6-(1-piperidinyl)-2,4-pyrimidinediamine-3-oxide, finasteride, ketoconazole, zinc pyrithione, coal tar, benzoyl peroxide, selenium sulfide, hydrocortisone, sulfur, menthol, pramoxine hydrochloride, tricetylmonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolyzed wheat proteins, hydrolyzed silk proteins, octyl methoxycinnamate, oxybenzone, minoxidil, titanium dioxide, zinc dioxide, retinol, erthromycin, tretinoin, and mixtures thereof.

One preferred type of benefit agent includes those therapeutic components that are effective in the treatment of seborrheic dermatitis, and psoriasis as well as the symptoms associated therewith. Examples of such suitable benefits agents nonexclusively include zinc pyrithione, anthralin, shale oil and derivatives thereof such as sulfonated shale oil, selenium sulfide, sulfur; salicylic acid; coal tar; povidone-iodine, imidazoles such as ketoconazole, dichlorophenyl imidazolodioxalan, which is commercially available from Janssen Pharmaceutica, N.V., under the tradename, "Elubiol", clotrimazole, itraconazole, miconazole, climbazole, tioconazole, sulconazole, butoconazole, fluconazole, miconazole nitrate and any possible stereo isomers and derivatives thereof; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, e.g. vitamin A palmitate, retinoids, retinols, and retinoic acid; corticosteroids such as hydrocortisone, clobetasone, butyrate, clobetasol propionate and mixtures thereof.

The amount of benefit agent to be combined with the composition or the emulsion of this invention may vary depending upon, for example, the ability of the benefit agent to penetrate through the skin, hair or nail, the specific benefit agent chosen, the particular benefit desired, the sensitivity of the user to the benefit agent, the health condition, age, and skin, hair, and/or nail condition of the user, and the like. In sum, the benefit agent is used in a "safe and effective amount," which is an amount that is high enough to deliver a desired skin, hair or nail benefit or to modify a certain condition to be treated, but is low enough to avoid serious side effects, at a reasonable risk to benefit ratio within the scope of sound medical judgment.

Examples of suitable anti-aging agents include, but are not limited to inorganic sunscreens such as titanium dioxide and zinc oxide; organic sunscreens such as octyl-methoxy cinnamates and derivatives thereof; retinoids; vitamins such as vitamin E, vitamin A, vitamin C, vitamin B, and derivatives thereof such as vitamin E acetate, vitamin C palmitate, and the like; beta hydroxy acids such as beta-hydroxybutyric acid, beta-phenyl-lactic acid, beta-phenylpyruvic acid; botanical extracts such as green tea, soy, milk thistle, algae, *aloe,* angelica, bitter orange, coffee, goldthread, grapefruit, hoellen, honeysuckle, Job's tears, lithospermum, mulberry, peony, puerarua, nice, safflower, and mixtures thereof.

Preferred anti-aging agents include retinoids, anti-oxidants, alpha-hydroxy acids and beta-hydroxy acid with retinol and tretinoin being most preferred.

Examples of suitable anti-acne agents include known to those of skill in the art work of those compositions.

Preferred anti-acne agents include benzoyl peroxide, retinol, elubiol, antibiotics, and salicylic acid, with retinol and tretinoin being most preferred.

Examples of benefit agents suitable for treating the symptoms and/or the diseases of seborrheic dermatitis and/or psoriasis, respectively, nonexclusively include those set forth above with shale oil and derivatives thereof, elubiol, ketoconazole, coal tar, zinc pyrithione, selenium sulfide, hydrocortisone, sulfur, menthol, pramoxine hydrochloride, and mixtures thereof being particularly preferred.

The compositions of the present invention may be directed applied to the skin or may be applied onto other delivery implements such as wipes, sponges, brushes, and the like. The compositions may be used in products designed to be left on the skin, wiped from the skin, or rinsed off of the skin.

Skin mildness of the compositions of this invention may be measured using the EpiDerm-ET50 test. This test consists of the determination of the pH of the neat liquid test article if possible (and/or dosing solution as appropriate) and a definitive assay to determine the ET50 (the exposure time which reduces MTT reduction by 50%). The toxicity of the test article is evaluated on the basis of the relative tissue viability versus exposure time. Viability will be determined by the NAD(P)H-dependent microsomal enzyme reduction of MTT (and to a lesser extent, by the succinate dehydrogenase reduction of MTT) in control and test article-treated cultures (Berridge, et al., 1996). Data are presented in the form of relative survival (relative MTT conversion) versus exposure time. Preferably, the skin mildness scores of the compositions and methods of this invention should be greater than about 10 hours, more preferably greater than about 15 hours and most preferably greater than about 20 hours.

Eye mildness of the compositions of this invention may be measured using the EpiOcular-ET50 test. This test consists of a determination of the direct MTT reduction potential and pH of the neat liquid test article if possible (and/or dosing solution as appropriate) and a single definitive asay. The toxicity of the test article will be evaluated by the exposure time required to reduce tissue viability to 50% of controls (ET50). Viability will be determined by the NAD(P)H-dependent microsomal enzyme reduction of MTT (and to a lesser extent, by the succinate dehydrogenase reduction of MTT) in control and test article-treated cultures (Berridge, et al., 1996.) Data will be presented in the form of relative survival (relative MTT conversion) versus test article exposure time. Preferably, the eye mildness scores of the compositions and methods of this invention should be greater than about 10 hours more preferably greater than about 14 hours and most preferably greater than about 15 hours.

The methods and compositions of this invention illustratively disclosed herein suitably may be practiced in the absence of any component, ingredient, or step which is not specifically disclosed herein. Several examples are set forth below to further illustrate the nature of the invention and the manner of carrying it out. However, the invention should not be considered as being limited to the details thereof.

**Example 1**

A composition in accordance with the compositions of this invention having the following ingredients and amounts was prepared:

Water phase

| Function | Ingredient | % w/w |
|---|---|---|
| Vehicle | Deionized Water | 82.90 |
| Thickener | Xanthan Gum | 0.03 |
| Thickener | Cetyl Hydroxyethylcellulose | 0.05 |
| Humectant | Glycerin | 4.00 |

The vehicle was added to the main beaker. A thickener was added to the water and mixed until completely solubilized. An additional thickener was then added and completely dispersed. The solution was heated to 70-75° C while continuously mixing. The humectant was added and mixed until homogeneous.

Oil Phase

| Function | Ingredient | % w/w |
|---|---|---|
| Emulsifier | Candelilla/Jojoba/Rice Bran Polyglycerol-3 Esters (and) Glyceryl Stearate (and) Cetearyl Alcohol (and) Sodium Stearoyl Lactylate | 3.00 |
| Emollient | Glycine Soja (Soybean) Oil (and ) Hydrogenated Cottonseed Oil | 3.00 |
| Thickener | Cetyl Alcohol | 1.00 |
| Emollient | Cocoglycerides | 2.00 |
| Emollient | Glyceryl Laurate | 3.00 |
| Preservative | Benzoic Acid | 0.30 |
| | Total | 100.00 |

In a separate beaker, the emulsifier, emollients, and thickener were added. Mixing was started while heating the composition to 70-75° C. When the oil phase reached 60-65° C, the benzoic acid was added. The composition was mixed until the ingredients were completely dissolved and the temperature reached 70-75°C.

**Post Phase**

When both phases were 70 to about 75 C, the oil phase was added to the water and mixed. The resultant solution was allowed to cool to room temperature.

**Example 2 - Working Example**

| Function | Ingredient | %w/w |
|---|---|---|
| Vehicle | Water | 85.30 |
| Thickener | Xanthan Gum | 0.20 |
| Thickener | Cetyl Hydroxyethylcellulose | 0.50 |
| Humectant | Glycerin | 3.00 |
| Emulsifier | Glyceryl stearate | 1.50 |
| Emulsifier | Hydrogenated Olive Oil (and) Olive Oil (and) Olive Oil Unsaponifiable | 1.50 |
| Emollient | Shea Butter | 1.50 |
| Emollient | Glyceryl Laurate | 3.00 |
| Emollient | Cocoglycerides | 3.00 |
| Preservative | Benzoic Acid | 0.50 |
| | Total | 100.00 |

Water is added to a beaker and mixing begun. The thickener is added to the beaker and the ingredients mixed until they are completely dispersed. Heating to 75 to 80°C is begun. Humectant is added and mixing continued. When the water phase reached 75 to 80°C, the temperature is held while mixing is continued. Separately, the oil phase is created. Emulsifiers, emollients and preservative are added to another beaker and mixing begun while the oil phase is heated. When the oil phase temperature reaches 75 to 80°C, the oil phase is added to the water phase. The batch is then cooled and the mixing speed decreased.

**Example 3**

| | | |
|---|---|---|
| Vehicle | Water | 70.74 |
| Humectant | Oatmeal | 1.00 |
| Thickener | Sodium Chloride | 0.01 |
| Emulsifier | Distearyldimonium chloride | 5.00 |
| Thickener | Cetyl Alcohol, NF | 2.50 |
| Emollient | Dimethicone | 1.25 |
| Emollient | Petrolatum, USP | 4.00 |
| Emollient | Isopropyl Palmitate | 3.00 |
| Humectant | Glycerin, USP | 12.00 |
| Preservative | Benzoic Acid | 0.50 |
| | **TOTAL** | 100.00 |

A premix of emollients was made by adding petrolatum, dimethicone and benzoic acid to a separate beaker and the premix was heated to 75 to 77°C. The premix was mixed until all chemicals were melted and held for addition to the other ingredients in a main batch. The water phase was made by adding water to a main tank and mixing begun. Oatmeal and sodium chloride were added and mixed until completely dispersed. Heating was begun to 75-77°C. During the heating process, emulsifier and thickener were added. When the batch reached a temperature between 75-77°C, the emollient premix was added to the main tank. The premix vessel was rinsed with pisopropyl palmitate. The temperature of the main tank was maintained while mixing for three to four minutes. The main batch was cooled to 25-27°C and glycerin added.

**Example 4**

| Function | Ingredient | % w/w |
|---|---|---|
| Vehicle | Water | 76.19 |
| Thickener | Carbopol | 0.37 |
| Humectant | Glceyrin | 10.00 |
| Preservative | Benzoic Acid | 0.30 |
| Emulsifier | Potassium Cetyl phosphate; hydrogenated palm glycerides | 0.60 |
| Emollient | Glycine Soja Oil; Hydrogenated Cottonseed Oil | 4.00 |
| Emollient | Cocoglycerides | 2.00 |
| pH Adjusting Agent | Sodium Hydroxide | 0.04 |
| Humectant | Corn Starch | 1.00 |
| | Total | 100.00 |

**Water Phase:** Vehicle was added to the main tank and mixing begun. Thickener was added to the vehicle and mixed until completely dispersed. The water phase was then neutralized with the pH adjusting agent.

**Oil Phase:** In a separate tank, emulsifier, preservative and emollients were added. Heating to 80-85°C was begun. When both the phases were at a temperature of 80-85°C, the oil phase was added to the water phase and mixed until homogeneous. The mixture was cooled to about 30°C, at which time corn starch was added and mixing continued until the mixture was homogeneous.

**Example 5 - Buffer Capacity**

Buffer capacity of the compositions of Example 1 and certain comparative compositions was determined using the following method:

Three grams of the test lotion was weighted into a 150 mL Erlemeyer flask. 25 mL of deionized water was added and the resulting composition was mixed until uniform. Three drops of color indicator solution, phenolphthalein, was added and the composition mixed. 0.1N sodium hydroxide was then titrated into the composition until a color change was observed. The amount of sodium hydroxide used was noted and the buffer capacity, expressed in units, calculated using the following equation.

$$\beta = 2.303\left(\frac{K_w}{[H^+]} + [H^+] + \Sigma \frac{C_{buf} K_a[H^+]}{K_a + [H^+]^2}\right)$$

Where:

$C_{buf}$ = concentration of buffer

$K_w$ = water ionization constant

$K_\alpha$ = acid dissociation constant

$\beta$ = buffer capacity

**pH Measurements**

pH measurements were performed over the period of a week using a Corning Pinnacle model 530 pH electrode with a Corning 3 in 1 Combo RJ electrode. The pH meter was calibrated daily.

| Sample Name | pH | Buffer Capacity (units) |
|---|---|---|
| Comparative Example 1 | 3.82 | 0.01 |
| Comparative Example 2 | 5.92 | 0.51 |
| Comparative Example 3 | 2.86 | 0.04 |
| Comparative Example 4 | 4.66 | 0.02 |
| Comparative Example 5 | 4.85 | 0.35 |
| Comparative Example 6 | 5.81 | 0.01 |
| Inventive Sample 1 | 4.8 | 0.019 |

**Comparative Example 1** - Baby Magic Baby Lotion (Contains Benzalkonium chloride, methylparaben, propylparaben, diazolidinyl urea; available from Naterra International, Inc.; Flower Mound, TX 75028)
**Comparative Example 2** - Jason Earth's Best Everyday Lotion (Contains Benzyl alcohol, potassium sorbate, sodium benzoate; available from JASON NATURAL PRODUCTS; Culver City, CA 90232)
**Comparative Example 3** - California Baby Super Sensitive Lotion (Contains Polyaminopropyl biguanide; available from Honky Tots, Inc. dba California Baby®; Beverly Hills, CA 90212)
**Comparative Example 4** - Method Baby Body Lotion (Contains Dehydroacetic Acid, Benzyl Alcohol; available from Method Products, Inc.; San Francisco, CA 94111)
**Comparative Example 5** - Burt's Bees Buttermilk Lotion (Contains Glucose oxidase and lactoperoxidase; available from Burt's Bees, Inc. Durham, NC 27709)

**Comparative Example 6** -. Seba Med Baby Lotion (Contains Alcohol, benzyl alcohol, phenoxyethanol, sodium benzoate; available from Physician Laboratories; Scottsdale, AZ 85258)

**Example 6 - Mildness**

Skin mildness of the compositions set forth below was measured using the EpiDerm-ET50 test. Eye mildness of the compositions set forth below in Table I was measured using the EpiOcular-ET50 test. Skin mildness and eye mildness results are set forth below in Table I.

**Test Composition 1 (TC-1)**

Water phase

| Function | Ingredient | % w/w |
|---|---|---|
| Vehicle | Deionized Water | 82.70 |
| Thickener | Xanthan Gum | 0.03 |
| Thickener | Cetyl Hydroxyethylcellulose | 0.05 |
| Humectant | Glycerin | 4.00 |

The vehicle was added to the main beaker. A thickener was added to the water and mixed until completely solubilized. An additional thickener was then added and completely dispersed. The solution was heated to 70-75° C while continuously mixing. The humectant was added and mixed until homogeneous.

Oil Phase

| Function | Ingredient | % w/w |
|---|---|---|
| Emulsifier | Candelilla/Jojoba/Rice Bran Polyglycerol-3 Esters (and) Glyceryl Stearate (and) Cetearyl Alcohol (and) Sodium Stearoyl Lactylate | 3.00 |
| Emollient | Glycine Soja (Soybean) Oil (and ) Hydrogenated Cottonseed Oil | 3.00 |
| Thickener | Cetyl Alcohol | 1.00 |
| Emollient | Cocoglycerides | 2.00 |
| Emollient | Glyceryl Laurate | 3.00 |
| Preservative | Benzoic Acid | 0.50 |
| | Total | 100.00 |

In a separate beaker, the emulsifier, emollients, and thickener were added. Mixing was started while heating the composition to 70-75° C. When the oil phase reached 60-65° C, the benzoic acid was added. The composition was mixed until the ingredients were completely dissolved and the temperature reached 70-75°C.

**Test Composition 2 (TC-2):**

Water phase

| Function | Ingredient | % w/w |
|---|---|---|
| Vehicle | Deionized Water | 82.05 |
| Buffering Agent | Sodium Citrate | 0.20 |
| Thickener | Xanthan Gum | 0.03 |
| Thickener | Cetyl Hydroxyethylcellulose | 0.05 |
| Humectant | Glycerin | 4.00 |

The vehicle was added to the main beaker. The buffering agent was then added to the water phase and mixed until completely dispersed. A thickener was added to the water and mixed until completely solubilized. An additional thickener was then added and completely dispersed. The solution was heated to 70-75° C while continuously mixing. The humectant was added and mixed until homogeneous.

Oil Phase

| Function | Ingredient | % w/w |
|---|---|---|
| Emulsifier | Candelilla/Jojoba/Rice Bran Polyglycerol-3 Esters (and) Glyceryl Stearate (and) Cetearyl Alcohol (and) Sodium Stearoyl Lactylate | 3.00 |
| Emollient | Glycine Soja (Soybean) Oil (and ) Hydrogenated Cottonseed Oil | 3.00 |
| Thickener | Cetyl Alcohol | 1.00 |
| Emollient | Cocoglycerides | 2.00 |
| Emollient | Glyceryl Laurate | 3.00 |
| Preservative | Benzoic Acid | 0.50 |
| pH Adjusting Agent | Sodium Hydroxide (and) Water | 0.45 |
| | Total | 100.00 |

In a separate beaker, the emulsifier, emollients, and thickener were added. Mixing was started while heating the composition to 70-75° C. When the oil phase reached 60-65° C, the benzoic acid was added. The composition was mixed until the ingredients were completely dissolved and the temperature reached 70-75°C.

Post Phase

The pH was then adjusted to 6.9 with 10% sodium hydroxide solution.

**Test Composition 3 (TC-3):**

Water phase

| Function | Ingredient | % w/w |
|---|---|---|
| Vehicle | Deionized Water | 82.50 |
| Buffering Agent | Sodium Citrate | 0.20 |
| Thickener | Xanthan Gum | 0.03 |
| Thickener | Cetyl Hydroxyethylcellulose | 0.05 |
| Humectant | Glycerin | 4.00 |

The vehicle was added to the main beaker. The buffering agent was then added to the water phase and mixed until completely dispersed. A thickener was added to the water and mixed until completely solubilized. An additional thickener was then added and completely dispersed. The solution was heated to 70-75° C while continuously mixing. The humectant was added and mixed until homogeneous.

Oil Phase

| Function | Ingredient | % w/w |
|---|---|---|
| Emulsifier | Candelilla/Jojoba/Rice Bran Polyglycerol-3 Esters (and) Glyceryl Stearate (and) Cetearyl Alcohol (and) Sodium Stearoyl Lactylate | 3.00 |
| Emollient | Glycine Soja (Soybean) Oil (and ) Hydrogenated Cottonseed Oil | 3.00 |
| Thickener | Cetyl Alcohol | 1.00 |
| Emollient | Cocoglycerides | 2.00 |
| Emollient | Glyceryl Laurate | 3.00 |
| Preservative | Benzoic Acid | 0.50 |
| | Total | 100.00 |

In a separate beaker, the emulsifier, emollients, and thickener were added. Mixing was started while heating the composition to 70-75° C. When the oil phase reached 60-65° C, the benzoic acid was added. The composition was mixed until the ingredients were completely dissolved and the temperature reached 70-75°C.

**Test Composition 4 (TC-4):**

Water phase

| Function | Ingredient | % w/w |
|---|---|---|
| Vehicle | Deionized Water | 82.20 |
| Thickener | Xanthan Gum | 0.03 |
| Thickener | Cetyl Hydroxyethylcellulose | 0.05 |
| Humectant | Glycerin | 4.00 |
| Preservative | Sodium Benzoate | 0.50 |

The vehicle was added to the main beaker. A thickener was added to the water and mixed until completely solubilized. An additional thickener was then added and completely dispersed. The solution was heated to 70-75° C while continuously mixing. The humectant was added and mixed until homogeneous. The preservative was added and mixed until completely dispersed.

Oil Phase

| Function | Ingredient | % w/w |
|---|---|---|
| Emulsifier | Candelilla/Jojoba/Rice Bran Polyglycerol-3 Esters (and) Glyceryl Stearate (and) Cetearyl Alcohol (and) Sodium Stearoyl Lactylate | 3.00 |

(continued)

| Function | Ingredient | % w/w |
|---|---|---|
| Emollient | Glycine Soja (Soybean) Oil (and ) Hydrogenated Cottonseed Oil | 3.00 |
| Thickener | Cetyl Alcohol | 1.00 |
| Emollient | Cocoglycerides | 2.00 |
| Emollient | Glyceryl Laurate | 3.00 |
| pH Adjusting Agent | Citric Acid (and) Water | 0.5 |
| | Total | 100.00 |

In a separate beaker, the emulsifier, emollients, and thickener were added. Mixing was started while heating the composition to 70-75° C. The composition was mixed until the ingredients were completely dissolved and the temperature reached 70-75° C.

Post Phase

The pH was then adjusted to 4.8 using a 20% citric acid solution.

**Test Composition 5 (TC-5:**

Water phase

| Function | Ingredient | % w/w |
|---|---|---|
| Vehicle | Deionized Water | 82.30 |
| Thickener | Xanthan Gum | 0.03 |
| Thickener | Cetyl Hydroxyethylcellulose | 0.05 |
| Humectant | Glycerin | 4.00 |
| Preservative | Sodium Benzoate | 0.50 |

The vehicle was added to the main beaker. A thickener was added to the water and mixed until completely solubilized. An additional thickener was then added and completely dispersed. The solution was heated to 70-75° C while continuously mixing. The humectant was added and mixed until homogeneous. The preservative was added and mixed until completely dispersed.

Oil Phase

| Function | Ingredient | % w/w |
|---|---|---|
| Emulsifier | Candelilla/Jojoba/Rice Bran Polyglycerol-3 Esters (and) Glyceryl Stearate (and) Cetearyl Alcohol (and) Sodium Stearoyl Lactylate | 3.00 |
| Emollient | Glycine Soja (Soybean) Oil (and ) Hydrogenated Cottonseed Oil | 3.00 |
| Thickener | Cetyl Alcohol | 1.00 |
| Emollient | Cocoglycerides | 2.00 |
| Emollient | Glyceryl Laurate | 3.00 |
| pH Adjusting Agent | Citric Acid (and) Water | 0.40 |

(continued)

| Function | Ingredient | % w/w |
|---|---|---|
|  | Total | 100.00 |

In a separate beaker, the emulsifier, emollients, and thickener were added. Mixing was started while heating the composition to 70-75° C. The composition was mixed until the ingredients were completely dissolved and the temperature reached 70-75° C.

Post Phase

The pH was then adjusted to 5.0 using a 20% citric acid solution.

**Table I**

| Formula # | Description | pH (at time of manufacture) | Buffer Capacity (units) | Eye Mildness EpiOcular -ET50 (hours) | Skin Mildness EpiDerm -ET50 (hours) |
|---|---|---|---|---|---|
| TC-1 | 0.5% Benzoic Acid, No Buffer | 4.4 | 0.019 | 17.4 | >24 |
| Example 1 | 0.3% Benzoic Acid, No Buffer | 4.8 | 0.023 | 15.4 | 22.9 |
| TC-2 | 0.5% Benzoic Acid with sodium citrate buffer | 6.9 | 0.028 | 16.4 | >24 |
| TC-3 | 0.5% Benzoic Acid with sodium citrate buffer | 4.7 | 0.031 | 20.4 | >24 |
| TC-4 | 0.5% Sodium Benzoate | 4.8 | 0.030 | 15.7 | 17.7 |
| TC-5 | 0.5% Sodium Benzoate with sodium citrate buffer | 5.0 | 0.050 | 19.1 | 14 |

Thus, it can be seen that the compositions of this invention are mild to the skin and eyes. It is theorized that the lower the buffer capacity, the milder the compositions are with respect to the eyes and skin. TC-1, containing benzoic acid but no buffer, has a lower buffer capacity and higher EpiOcular score than that of TC-2, which contains both benzoic acid and a sodium citrate buffer and which has a higher pH (6.9). TC-5, containing sodium benzoate and a sodium citrate buffer, and which has the highest buffer capacity of the formulations in Table I, had the lowest EpiDerm score. This indicates it would be the least mild to the skin. TC-2 had a high pH as well as high EpiOcular and EpiDerm scores, indicating higher mildness. However, the benzoic acid preservative in TC-2 is present as sodium benzoate and would be ineffective as a preservative in that form at that pH.

**Claims**

1. A buffered leave-on skin care composition comprising: a cosmetically acceptable oil; water; a cosmetically acceptable emulsifier having an HLB of from about 1 to about 25; and a preservative comprising an organic acid selected from the group consisting of benzoic acid, p-anisic acid, sorbic acid, lactic acid, acetic acid, formic acid, oxalic acid, tartaric acid, salicylic acid and citric acid; wherein said composition has a pH less than 5 and a buffer capacity of between about 0.001 and about 0.039.

2. A composition according to Claim 1, wherein said organic acid is benzoic acid.

3. A composition according to Claim 1 or Claim 2, wherein the pH is less than about 4.8.

4. A composition according to any preceding Claim, wherein the buffer capacity is from 0.001 to about 0.031; or from about 0.001 to about 0.023.

5. A composition according to any preceding Claim wherein the skin mildness score of the composition is greater than about 10.

6. A composition according to any preceding Claim wherein the eye mildness score of the composition is greater than about 10.

7. A method of preserving a leave-on skin care composition comprising combining a cosmetically acceptable oil; a preservative comprising an organic acid selected from the group consisting of benzoic acid, p-anisic acid, sorbic acid, lactic acid, acetic acid, formic acid, oxalic acid, tartaric acid, salicylic acid and citric acid; a cosmetically acceptable emulsifier having an HLB of from about 1 to about 25; wherein said composition has a pH less than 5 and a buffer capacity of between about 0.001 and about 0.039.

8. A method according to Claim 7, wherein said organic acid is benzoic acid.

9. A method according to Claim 7 or Claim 8, wherein the pH is less than about 4.8.

10. A cosmetically acceptable wipe comprising a nonwoven substrate and a buffered leave-on skin care composition of between about 0.001 and about 0.039 as Claimed in any one of Claims 1 to 6.

11. A method of providing moisture to mammalian skin comprising applying to said skin the leave-on skin care composition according to any one of claims 1 to 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3755560 A
- US 4421769 A
- US 4254105 A
- US 4960764 A
- US 6063397 A

**Non-patent literature cited in the description**

- McCutcheon's Detergents and Emulsifiers. 1986, 317-324
- ICI Handbook. 1673-1686